# EUROPEAN PATENT APPLICATION

(11) **EP 3 290 562 A1**
(43) Date of publication of application: **07.03.2018**
(21) Application number: 16785837.2
(22) Date of filing: 15.04.2016
(51) Int. Cl.: D06F 35/00

(54) **OZONE STERILIZATION WASHING MACHINE AND METHOD**

(30) Priority: 28.04.2015 CN 201510208615
(71) Applicant: Qingdao Haier Drum Washing Machine Co., Ltd., Qingdao, Shandong 266101 (CN)
(72) Inventor: XU, Sheng, Qingdao Shandong 266101 (CN); ZHAO, Zhiqiang, Qingdao Shandong 266101 (CN); LV, Yanfen, Qingdao Shandong 266101 (CN)
(74) Representative: Ziebig, Marlene
(86) International application number: PCT/CN2016/079400
(87) International publication number: WO 2016/173406

(57) **Abstract**

Provided are an ozone sterilization washing machine and a method. The ozone sterilization washing machine comprises an outer barrel (1), an ozone generation device (2) and a heating device (3), wherein an air inlet communicated with the ozone generation device (2) is arranged at the bottom of the outer barrel (1), an exhaust hole is arranged at the upper part of the outer barrel (1) and communicated with an exhaust pipeline (10), and the heating device (3) is arranged in the exhaust pipeline (10) to decompose ozone. According to the ozone sterilization washing machine, clothes are sterilized with ozone, and ozone which is not completely dissolved is thoroughly decomposed under a high temperature at a high decomposition rate; in addition, no catalyst is added, thus saving manpower and material resources.

## Description

### Technical Field

The present invention relates to an ozone sterilization washing machine and a method.

### Background

Washing machines bring a lot of convenience for people and have been widely used in the family; however, when washing machines are used to wash clothes, breading of a lot of bacteria will be caused. In order to avoid the interactive infection of bacteria, microorganisms and parasites, some sterilization washing machines have come out and are mainly developed on the basis of UV sterilization, chemical fumigation and ozone sterilization, among which ozone sterilization with advantages of strong bactericidal ability, no residue, no damage to clothing and the like is most popular. Since ozone is hardly dissolved in water after being introduced in water, most of the ozone directly enters the barrel from the water and then is discharged, and inhaling too much ozone is harmful to human health.

The existing washing machine treats ozone in the following ways: a filter is arranged in the washing machine, the filter device is equipped with an activated carbon block, and ozone is decomposed through a chemical reaction between the activated carbon and ozone; or the structure of the filter device is changed to have a jagged outflowing path embedded in the filter device to speed up the natural decomposition of ozone.

The prior art has shortcomings of increase of the manpower and material resources due to addition of chemical raw materials into the filter device, too low decomposition rate and low practicality.

The present invention is provided in view of this.

### Summary of the Invention

The technical problem to be solved by the present invention is to overcome the shortcomings of the prior art and provide an ozone sterilization washing machine and a method, having the advantage of decomposing the incompletely dissolved ozone rapidly and thoroughly after sterilizing clothes with ozone.

The technical solution adopted by the present invention to solve its technical problem is as follows:
An ozone sterilization washing machine comprises an outer barrel, an ozone generation device and a heating device. An air inlet communicated with the ozone generation device is arranged at the bottom of the outer barrel, an exhaust hole is arranged at the upper part of the outer barrel and is communicated with an exhaust pipeline, and the heating device is arranged in the exhaust pipeline to decompose ozone.

Further, a temperature sensor for sensing air temperature is arranged in the heating device.

Further, the heating device operates at 80° C-300° C, preferably at 100° C-270° C.

Further, a draught fan is arranged in the exhaust pipeline.

Further, an ozone concentration detector for detecting ozone content in the air is arranged in the outer barrel.

Further, a one-way valve is arranged inside an air inlet pipeline.

The present invention further provides an ozone treatment method for the ozone sterilization washing machine, comprising:
Step S1: starting the ozone generation device at the beginning of a washing process;
Step S2: starting the heating device to decompose ozone;
Step S3: shutting down the ozone generation device at the end of the washing process; and
Step S4: shutting down the heating device.

Further, after the step S2, the method further comprises:
detecting in real time a temperature of air heated by the heating device, the heating device stopping heating when the temperature of the air is higher than a first preset temperature; when the temperature of the air is lower than a second preset temperature, the heating device being started for heating, wherein the first set temperature is higher than the second set temperature, and preferably, the first preset temperature is 150° C-300° C and the second preset temperature is 80° C-150° C.

Further, starting the heating device in the step S2 comprises: when the ozone concentration detector detects that the concentration of ozone in the outer barrel is higher than a preset concentration, starting the heating device; or
starting the heating device at the same time when the ozone generation device is started; or starting the heating device after the ozone generation device is started and kept running for a delay time T1.

Further, shutting down the heating device in the step S4 comprises: when the ozone concentration detector detects that the concentration of ozone in the air is lower than a second preset concentration, shutting down the heating device; or
shutting down the heating device while the ozone generation device is shut down; or
shutting down the heating device after the ozone generation device is shut down and kept closed for a delay time T2.

Further, the step S2 further comprises starting the draught fan when the heating device is started; and
the step S4 further comprises shutting down the draught fan when the heating device is shut down.

The technical solutions provided by the specific embodiments of the present invention have the following advantages:
Higher sterilization effect is achieved since clothes are sterilized with ozone; high decomposition rate and thorough decomposition effect are achieved because ozone which is not completely dissolved is decomposed under a high temperature and the temperature is controlled in real time; in addition, manpower and material resources are saved due to no need of a catalyst.

### Brief Description of the Drawings

The accompanying drawings, which are intended to be used in specific embodiments, will now be described briefly to make the technical solutions in the specific embodiments of the present invention more clear. It will be apparent that the drawings in the following description are merely of some embodiments of the present invention, and those skilled in the art also may obtain other drawings from these drawings without doing the creative work.
FIG. 1 is a schematic diagram of the structure of an ozone sterilization washing machine provided by the present invention;
FIG. 2 is a schematic diagram of the structure of another ozone sterilization washing machine provided by the present invention;
FIG. 3 is a flow chart of a method of an ozone sterilization washing machine provided by the present invention; and
FIG. 4 is a flow chart of another method of an ozone sterilization washing machine, provided by the present invention.

Legend: 1-outer barrel; 2-ozone generation device; 3-heating device; 4-one-way valve; 5-upward-draining pipeline; 6-downward-draining pipeline; 7-temperature sensor; 8-air inlet pipeline; 9-drain pipeline; 10-exhaust pipeline; 11-draught fan; P-drain pump/drain valve.

### Detailed Description of the Invention

### Embodiment 1:

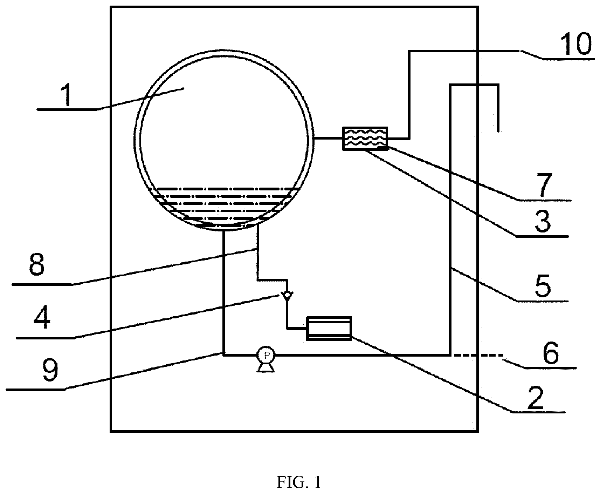

As shown in FIG. 1, the present invention provides an ozone sterilization washing machine, comprising an outer barrel 1, an ozone generation device 2 and a heating device 3.An air inlet communicated with the ozone generation device 2 is arranged at the bottom of the outer barrel 1, an exhaust hole is arranged at the upper part of the outer barrel 1 and is communicated with an exhaust pipeline 10, and the heating device 3 is arranged in the exhaust pipeline 10 to decompose ozone.

The ozone generation device 2 is used for generating ozone; ozone is introduced into water through the air inlet at the bottom of the outer barrel 1 and is dissolved in the water to kill bacteria; zone which is not dissolved in the water is discharged into the exhaust pipeline 10 through the exhaust hole at the upper part of the outer barrel 1 and is heated at a high temperature by the heating device 3 in the exhaust pipeline 10 and decomposed into oxygen, and then the oxygen is discharged out of the washing machine; in such a way, the ozone is rapidly decomposed.

A temperature sensor 7 is arranged in the heating device 3 and used for sensing the current temperature of air in real time. In order to implement high-temperature decomposition of ozone and increase the decomposition rate of ozone, the heating device 3 controls the temperature of heated air to be between a first preset temperature and a second preset temperature. In a washing process, after the heating device 3 is started for the first time, the following start and shutdown of the heating device 3 are related to the temperature detected by the temperature sensor 7. Specifically, in the operation of the heating device 3, when the air temperature is higher than the first preset temperature, the heating device 3 stops heating, and when the air temperature is lower than the second preset temperature, the heating device 3 is started. The first preset temperature is higher than the second preset temperature, and the heating device operates at 80° C-300° C, preferably at 100° C-270° C.

The principle of ozone decomposition is as follow. For ozone accounting for less than 1% in the air, its decomposition half-life in normal-state air at normal temperature and normal pressure is about 16 hours. With the rise of the temperature, the decomposition rate is increased, the decomposition becomes very dramatic when the temperature exceeds 100 ° C, and ozone can be immediately converted into oxygen when the temperature is as high as 270 ° C. Therefore, in order to accelerate the decomposition of ozone, the first preset temperature may be set between 150 ° C and 300 °C, preferably 270 °C, and the heating is stopped when the air temperature reaches 270 °C, thus ensuring rapid decomposition of ozone and preventing clothes from being damaged due to over high afterheat of air in the outer barrel 1. The second preset temperature is set between 80 °C and 150 °C, preferably 100 °C. When the air temperature is reduced to 100 °C, the heating device 3 is started for heating, thus, it can be avoided that the heating device 3 will be damaged if it is always in a working state, the rapid decomposition of ozone also can be ensured, and the temperature is controlled within an optimum range for ozone decomposition. In order to accommodate the temperature interval between the first preset temperature and the second preset temperature, the housing of the heating device 3 is configured as a metallic material, preferably an aluminum alloy material.

Preferably, an ozone concentration detector for detecting the ozone content in the air is arranged in the outer barrel 1. After the ozone generating device 2 is started, the first start of the heating device 3 may occur in the following cases:
Case 1: start the heating device 3 when the ozone concentration detector detects that the concentration of ozone in the outer barrel 1 is higher than a preset concentration;
Case 2: start the heating device 3 at the same time when the ozone generation device 2 is started; and
Case 3: start the heating device after the ozone generation device is started and kept running for a delay time T1.

Further, a one-way valve 4 is arranged in the air inlet pipeline 8 and is used to prevent water from flowing back into the ozone generation device 2 to damage the device and ensure that the ozone generated by the ozone generation device 2 is conveyed to the air inlet and introduced into the water.

Further, a water outlet is further arranged at the bottom of the outer barrel 1 and communicated with the drain pipeline 9. When the drainage mode of the washing machine is upward-draining, the drain pipeline 9 is an upward-draining pipeline, indicated by the reference numeral 5. A drain pump indicated by P in the drawing, is arranged in the upward-draining pipeline 5, and water is drained out of the washing machine through the upward-draining pipeline 5 after being pressurized by the drain pump. When the drainage mode of the washing machine is downward-draining, the drain pipeline 9 is a downward-draining pipeline, indicated by the reference numeral 6. A drain pump or drain valve indicated by P in the drawing, is arranged in the downward-draining pipeline 6, and water is drained out of the washing machine through the downward-draining pipeline 6 after being pressurized by the draining water or after the drain valve is opened.

The ozone sterilization washing machine in the present invention generates ozone by the ozone generation device 2, dissolves the ozone in water to sterilize washed clothes, and discharges the undissolved ozone into the heating device 3 through the exhaust hole, so that the ozone decomposition rate is increased by heating. In addition, the temperature sensor 7 in the heating device 3 detects the temperature in real time, and the washing machine controls the start and shutdown of the heating device 3 in accordance with the temperature change so as to control the air temperature in the exhaust pipeline 10 within the optimum range, thus realizing the rapid decomposition of ozone and achieving the thorough decomposition of ozone and increase of decomposition rate; in addition, due to no addition of a catalyst, manpower and material resources are saved.

### Embodiment 2:

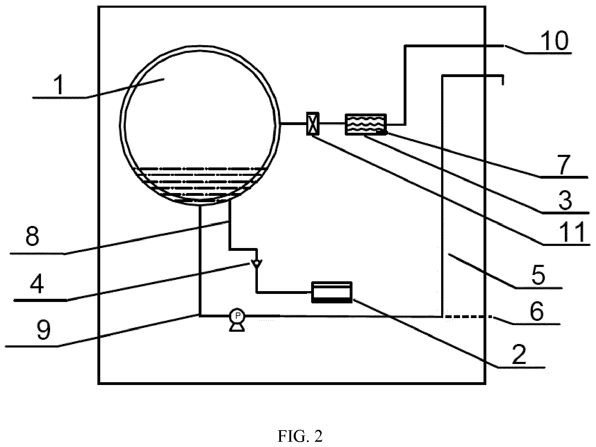

As shown in FIG. 2, this embodiment differs from Embodiment 1 in that a draught fan 11 is further arranged in the exhaust pipeline 10 and located between the exhaust hole and the heating device 3 to increase the rate at which ozone in an inner barrel flows towards the heating device 3, and the ozone reaches the heating device 3 for high-temperature decomposition, thereby achieving the purpose of increasing the decomposition rate of the ozone.

After the ozone generation device 2 is started, the draught fan is started along with the first start of the heating device 3 and operates continuously during the washing process. At the end of the washing process, after the ozone generator 2 is shut down, the draught fan 11 is shut down along with the shutdown of the heating device 3.

### Embodiment 3

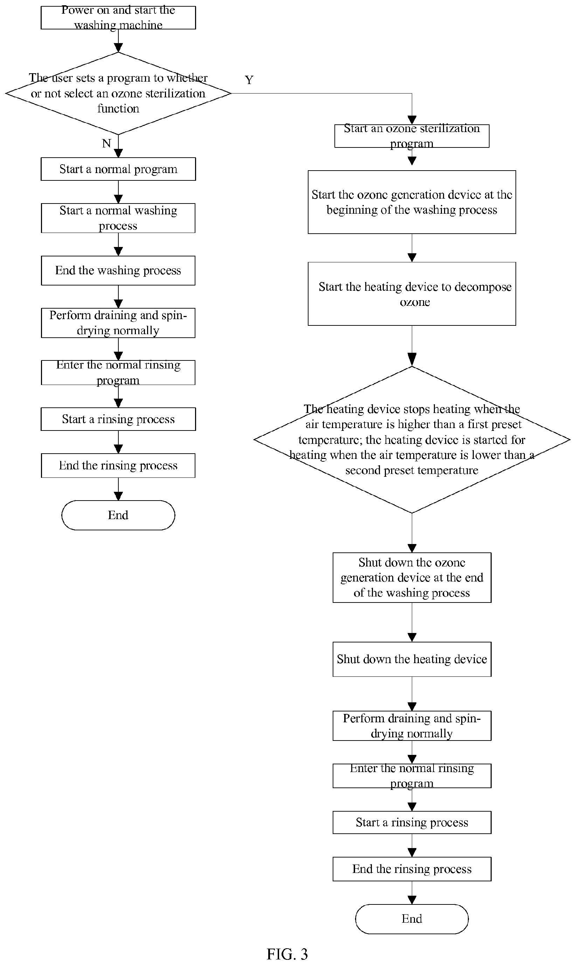

This embodiment provides a washing process performed by an ozone sterilization washing machine according to an ozone sterilization program. When the user does not select the ozone sterilization for washing, the washing machine performs a washing process according to a normal washing program, and when the user selects the ozone sterilization program for washing, the washing machine performs a washing process according to the ozone sterilization program. The executing subject of this embodiment is the washing machine; as shown in FIG. 3, a method of the washing machine performing an ozone sterilization program comprises:
301: Starting the ozone sterilization program.
302: Starting the ozone generation device at the beginning of a washing process.
302: Starting the heating device to decompose ozone.

Preferably, the condition of starting the heating device is: the heating device is started when the ozone concentration detector detects that the concentration of ozone in the outer barrel is higher than the preset concentration;

Preferably, the condition of starting the heating device is: the heating device is started at the same time when the ozone generation device is started;

Preferably, the condition of starting the heating device is: the heating device is started after the ozone generation device is started and kept running for a delay time T1, and T1 is preferably set within a range of 30s-2min.
303. Detecting in real time the temperature of air heated by the heating device, the heating device stopping heating when the air temperature is higher than a first preset temperature; the heating device being started for heating when the air temperature is lower than a second preset temperature, wherein the first set temperature is higher than the second set temperature.

Preferably, the first preset temperature is in a range of 150° C-300° C, further preferably 270° C, and the second preset temperature is in a range of 80° C-150° C, further preferably 100° C. The start and shutdown of the heating device is controlled by an internal program of the washing machine. The temperature sensed by the temperature sensor is transmitted in real time to the internal program of the washing machine, and then the internal program of the washing machine controls the start and shutdown of the heating device in real time according to the temperature.
304: Shutting down the ozone generation device at the end of the washing program.
305: Shutting down the heating device.

Preferably, the condition of shutting down the heating device is met: the heating device is shut down when the ozone concentration detector detects that the concentration of ozone in the air is lower than a second preset concentration;

Preferably, the condition of shutting down the heating device is met: the heating device is shut down while the ozone generation device is shut down;

Preferably, the condition of shutting down the heating device is met: the heating device is shut down after the ozone generation device is shut down and kept closed for a delay time T2, and T2 is preferably set within a range of 30s-5min.
306: Draining, spin-drying and rinsing normally.
307: Ending the washing process.

### Embodiment 4

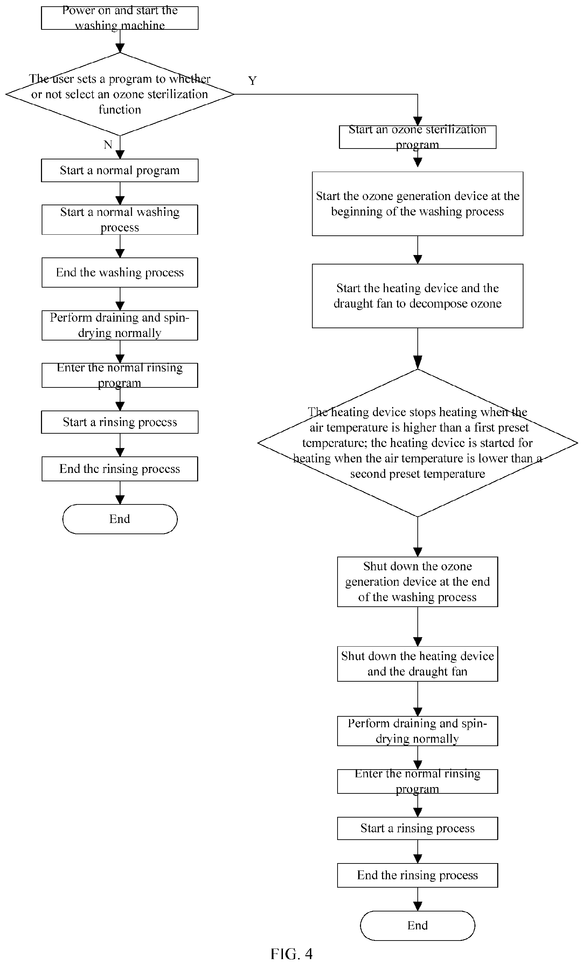

This embodiment provides a washing process performed by another ozone sterilization washing machine according to an ozone sterilization program. When the user does not select the ozone sterilization for washing, the washing machine performs a washing process according to a normal washing program, and when the user selects the ozone sterilization program for washing, the washing machine performs a washing process according to the ozone sterilization program. The executing subject of this embodiment is the washing machine; as shown in FIG. 4, a method of the washing machine performing an ozone sterilization program comprises:
401: Starting the ozone sterilization program.
402: Starting the ozone generation device at the beginning of a washing process.
402: Starting the heating device and the draught fan to decompose ozone.

Preferably, the condition of starting the heating device is met: the heating device and the draught fan are started when the ozone concentration detector detects that the concentration of ozone in the outer barrel is higher than the preset concentration;

Preferably, the condition of starting the heating device is met: the heating device and the draught fan are started while the ozone generation device is started;

Preferably, the condition of starting the heating device is: the heating device and the draught fan are started after the ozone generation device is started and kept running for a delay time T1, and T1 is preferably set within a range of 30s-2min.
403: Detecting in real time the temperature of air heated by the heating device, the heating device stopping heating when the air temperature is higher than a first preset temperature; the heating device being started for heating when the air temperature is lower than a second preset temperature, wherein the first set temperature is higher than the second set temperature.

Preferably, the first preset temperature is in a range of 150° C-300° C, further preferably 270° C, and the second preset temperature is in a range of 80° C-150° C, further preferably 100° C. The start and shutdown of the heating device is controlled by an internal program of the washing machine. The temperature sensed by the temperature sensor is transmitted in real time to the internal program of the washing machine, and then the internal program of the washing machine controls the start and shutdown of the heating device in real time according to the temperature.
404: Shutting down the ozone generation device at the end of the washing program.
405: Shutting down the heating device and the draught fan.

Preferably, the condition of shutting down the heating device is met: the heating device and the draught fan are shut down when the ozone concentration detector detects that the concentration of ozone in the air is lower than a second preset concentration;

Preferably, the condition of shutting down the heating device is met: the heating device and the draught fan are shut down while the ozone generation device is shut down;

Preferably, the condition of shutting down the heating device is met: the heating device and the draught fan are shut down after the ozone generation device is shut down and kept closed for a delay time T2, and T2 is preferably set within a range of 30s-5min.
406: Draining, spin-drying and rinsing normally.
407: Ending the washing process.

The foregoing is merely illustrative of the preferred embodiments of the present invention and is not intended to limit the present invention in any form. While the present invention has been disclosed as above by way of the preferred embodiments, it is not intended to be limiting of the present invention. Any person skilled in the art may make some changes or equivalent embodiments modified as equivalents without departing from the scope of the present invention on the basis of the above-mentioned technical contents; any of changes, equivalent, and modifications made to the foregoing embodiments without departing from the spirit of the technical solution of the present invention in accordance with the technical essence of the present invention is within the scope of the present invention.

## Claims

1. An ozone sterilization washing machine, comprising an outer barrel, an ozone generation device and a heating device, wherein an air inlet communicated with the ozone generation device is arranged at the bottom of the outer barrel, an exhaust hole is arranged at the upper part of the outer barrel and communicated with an exhaust pipeline, and the heating device is arranged in the exhaust pipeline to decompose ozone.

2. The ozone sterilization washing machine according to claim 1, wherein a temperature sensor for sensing air temperature is arranged in the heating device.

3. The ozone sterilization washing machine according to claim 1 or 2, wherein the heating device operates at a temperature range of 80° C-300° C, preferably at 100° C-270° C.

4. The ozone sterilization washing machine according to claim 1 or 2, wherein a draught fan is further arranged in the exhaust pipeline.

5. The ozone sterilization washing machine according to claim 1, wherein an ozone concentration detector for detecting ozone content in the air is arranged in the outer barrel.

6. The ozone sterilization washing machine according to claim 1, wherein an one-way valve is arranged in an air inlet pipeline.

7. An ozone treatment method for the ozone sterilization washing machine according to any of claims 1-6, comprising:
Step S1: starting the ozone generation device at the beginning of a washing process;
Step S2: starting the heating device to decompose ozone;
Step S3: shutting down the ozone generation device at the end of the washing process; and
Step S4: shutting down the heating device.

8. The ozone treatment method according to claim 7, wherein after the step S2, the method further comprises:
detecting in real time a temperature of air heated by the heating device, wherein, the heating device stops heating when the temperature of the air is higher than a first preset temperature, and the heating device is started for heating when the temperature of the air is lower than a second preset temperature, wherein the first set temperature is higher than the second set temperature, and
preferably, the first preset temperature is 150° C -300° C and the second preset temperature is 80° C-150° C.

9. The ozone treatment method according to claim 7, wherein a process for starting the heating device in the step S2 comprises: when the ozone concentration detector detects that the concentration of ozone in the outer barrel is higher than the preset concentration, starting the heating device; or
starting the heating device while the ozone generation device is started; or
starting the heating device after the ozone generation device is started and kept running for a delay time T1.

10. The ozone treatment method according to claim 7, wherein a process for shutting down the heating device in the step S4 comprises: when the ozone concentration detector detects that the concentration of ozone in the air is lower than the second preset concentration, shutting down the heating device; or
shutting down the heating device while the ozone generation device is shut down; or
shutting down the heating device after the ozone generation device is shut down and kept closed for a delay time T2.

11. The ozone treatment method according to any of claims 7-10, wherein the step S2 further comprises: starting the draught fan when the heating device is started;
the step S4 further comprises: shutting down the draught fan when the heating device is shut down.
